# EUROPÄISCHE PATENTANMELDUNG

(11) **EP 3 838 939 A1**
(43) Veröffentlichungstag der Anmeldung: **23.06.2021**
(21) Anmeldenummer: 19217953.9
(22) Anmeldetag: 19.12.2019
(51) Int. Cl.: C08G 18/02, C08G 18/20, C08G 18/79

(54) **KATALYSATORKOMPONENTE FÜR DIE ISOCYANATMODIFIZIERUNG**

(71) Anmelder: Covestro Deutschland AG, 51373 Leverkusen (DE)
(72) Erfinder: Die Erfindernennung liegt noch nicht vor
(74) Vertreter: Levpat

(57) **Zusammenfassung**

Die Erfindung betrifft eine Katalysatorkomponente für die Isocyanatmodifizierung, umfassend mindestens ein cyclisches Ammoniumsalz mit einem Kation der Formel I, 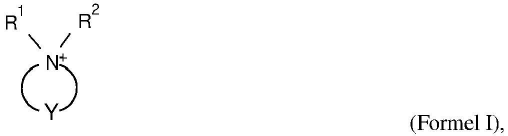 wobei Y für ein in 2-Stellung zum ladungstragenden Stickstoffatom durch eine Hydroxygruppe substituiertes und ggf. weitere Substituenten tragendes, lineares oder verzweigtes, ggf. durch Heteroatome aus der Reihe Sauerstoff, Schwefel, Stickstoff sowie aromatische Ringe unterbrochenes, ggf. weitere Ringe aufweisendes, C₂-C₂₀-Segment steht und die N-ständigen Substituenten R¹ und R² entweder unabhängig voneinander für gleiche oder verschiedene, substituierte oder unsubstituierte, ggf. verzweigte, aliphatische C₁-C₂₀ Reste, aromatische C₆-C₂₀ Reste oder aralipha¬tische C₇-C₂₀ Reste stehen oder die N-ständigen Substituenten R¹ und R² untereinander ein Ringsegment X bilden, für das die gleiche oder verschiedene vorstehend für Y gegebene Definition gilt, mit der Maßgabe, dass X eine in 2-Stellung zum ladungstragenden Stickstoffatom stehende Hydroxygruppe als Substituent aufweist oder keine in 2-Stellung zum ladungstragenden Stickstoffatom stehende Hydroxygruppe als Substituent aufweist.

## Beschreibung

Die Erfindung betrifft eine Katalysatorkomponente für die Isocyanatmodifizierung, sowie ein Verfahren zur Modifizierung von Isocyanaten in Gegenwart dieser Katalysatorkomponente. Außerdem betrifft die Erfindung modifizierte Isocyanate selbst und deren Verwendung zur Herstellung von Polyurethankörpern oder Beschichtungen sowie die Polyurethankörper oder Beschichtungen selbst. Weiterhin betrifft die Erfindung Ein- oder Zwei-Komponenten-Systeme umfassend die modifizierten Isocyanate.

Die Oligo- bzw. Polymerisierung von Isocyanaten insbesondere unter Bildung von höhermolekularen Oligomerengemischen mit Uretdion- ("Dimer"), Isocyanurat- ("Trimer") und/oder Iminooxadiazindionstrukturen ("asymmetrisches Trimer") im Molekülgerüst ist seit langem bekannt. Wie vorstehend erkennbar, liegen der Oligo- bzw. Polymerisierung von Isocyanaten prinzipiell die gleichen chemischen Reaktionen zugrunde. Die Umsetzung einer kleineren Anzahl von Isocyanaten miteinander bezeichnet man als Oligomerisierung. Die Umsetzung einer größeren Anzahl von Isocyanaten bezeichnet man als Polymerisierung. Im Rahmen der vorliegenden Erfindung wird die vorstehend beschriebene Oligomerisierung bzw. Polymerisierung von Isocyanaten zusammenfassend als Isocyanatmodifizierung oder Modifizierung von Isocyanaten bezeichnet.

Enthalten die modifizierten Polyisocyanate freie NCO-Gruppen, die ggf. auch mit Blockierungsmitteln vorübergehend desaktiviert worden sein können, sind sie außerordentlich hochwertige Ausgangsstoffe für die Herstellung einer Vielzahl von Polyurethan-Kunststoffen und Beschichtungsmitteln.

Es haben sich eine Reihe technischer Verfahren zur Isocyanatmodifizierung etabliert, wobei man in der Regel das zu modifizierende Isocyanat, meist ein Diisocyanat, durch Zusatz von Katalysatoren umsetzt und diese anschließend, wenn der gewünschte Umsatzgrad des zu modifizierenden Isocyanates erreicht ist, durch geeignete Maßnahmen unwirksam macht (deaktiviert) und das erhaltene Polyisocyanat in der Regel vom nicht umgesetzten Monomer abtrennt. Eine Zusammenstellung dieser Verfahren des Standes der Technik findet sich in H. J. Laas et al., J. Prakt. Chem. 1994, 336, 185 ff.

Als Modifizierungskatalysatoren haben sich ionisch aufgebaute Verbindungen bewährt, da sie in sehr geringer Menge, relativ zum umzusetzenden Monomer, eingesetzt werden können und äußerst schnell zum gewünschten Ergebnis führen, wobei die Kationen insbes. unter dem Aspekt der Löslichkeit des jeweiligen Salzes im Isocyanatmilieu von Bedeutung sind.

WO 2015/124504 A1 und WO 2017/029266 A1 beschreiben sehr stabile Ammoniumsalze, bei denen das ladungstragende Stickstoffatom Teil eines Ringsystems ist. Diese Verbindungen weisen allerdings den Nachteil auf, in isocyanatfunktionelle Verbindungen nicht einbaubar zu sein und daher als migrationsfähige Verunreinigungen im finalen Verfahrensprodukt, beispielsweise einem Polyurethan-Kunststoff oder einer Polyurethanbeschichtung, zu verbleiben und später zu unerwünschten Effekten wie fogging etc. Anlass zu geben.

Der Erfindung lag daher die Aufgabe zugrunde, eine Katalysatorkomponente für die Isocyanatmodifizierung bereitzustellen, die aus preiswerten Edukten leicht herstellbar sind und eine hohe katalytische Aktivität und Selektivität bei gleichzeitig guter Katalysatorstabilität und geringer Neigung zur Bildung störender Nebenprodukte in Verfahren zur Isocyanatmodifizierung aufweisen.

Diese Aufgabe wurde erfindungsgemäß gelöst durch eine Katalysatorkomponente für die Isocyanatmodifizierung, umfassend mindestens ein cyclisches Ammoniumsalz mit einem Kation der Formel I, 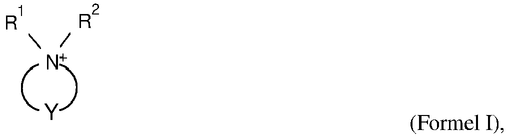 wobei
Y für ein in 2-Stellung zum ladungstragenden Stickstoffatom durch eine Hydroxygruppe substituiertes und ggf. weitere Substituenten tragendes, lineares oder verzweigtes, ggf. durch Heteroatome aus der Reihe Sauerstoff, Schwefel, Stickstoff sowie aromatische Ringe unterbrochenes, ggf. weitere Ringe aufweisendes, C₂-C₂₀-Segment steht und
die N-ständigen Substituenten R¹ und R² entweder unabhängig voneinander für gleiche oder verschiedene, substituierte oder unsubstituierte, ggf. verzweigte, aliphatische C₁-C₂₀ Reste, aromatische C₆-C₂₀ Reste oder araliphatische C₇-C₂₀ Reste stehen oder
die N-ständigen Substituenten R¹ und R² untereinander ein Ringsegment X bilden, für das die gleiche oder verschiedene vorstehend für Y gegebene Definition gilt, mit der Maßgabe, dass X eine in 2-Stellung zum ladungstragenden Stickstoffatom stehende Hydroxygruppe als Substituent aufweist oder keine in 2-Stellung zum ladungstragenden Stickstoffatom stehende Hydroxygruppe als Substituent aufweist.

Bevorzugt bedeuten die Bezugnahmen auf "umfassend", "enthaltend" usw. "im Wesentlichen bestehend aus" und ganz besonders bevorzugt "bestehend aus". Die in den Patentansprüchen und in der Beschreibung genannten weiteren Ausführungsformen können beliebig kombiniert werden, sofern sich aus dem Kontext nicht eindeutig das Gegenteil ergibt.

In einer ersten bevorzugten Ausführungsform steht Y für ein in 2-Stellung zum ladungstragenden Stickstoffatom durch eine Hydroxygruppe substituiertes und ggf. weitere Substituenten tragendes C₄-C₆-Alkylenkettensegment.

In einer weiteren bevorzugten Ausführungsform stehen R¹ und R² unabhängig voneinander für gleiche oder verschiedene C₁-C₈-Alkylsubstituenten oder für gleiche oder verschiedene, ggf. am aromatischen Kern substituierte, Benzyl-Reste, bevorzugt für gleiche oder verschiedene C₁-C₆-Alkylsubstituenten und besonders bevorzugt für gleiche oder verschiedene, linear aufgebaute C₁-C₆-Alkylsubstituenten.

In einer weiteren bevorzugten Ausführungsform bilden R¹ und R² untereinander ein Ringsegment X, wobei X ein ggf. weitere Substituenten tragendes C₄-C₆-Alkylenkettensegment ist, mit der Maßgabe, dass X in 2-Stellung zum ladungstragenden Stickstoffatom durch eine Hydroxygruppe substituiert ist oder nicht durch eine Hydroxygruppe in 2-Stellung zum ladungstragenden Stickstoffatom substituiert ist.

In einer weiteren bevorzugten Ausführungsform sind das Segment Y und/oder das Ringsegment X linear aufgebaut.

In einer weiteren bevorzugten Ausführungsform weist nur Y eine Hydroxygruppe auf.

Kationen der Formel I in denen die N-ständigen Substituenten R¹ und R² untereinander ein Ringsystem bilden, stellen spirocyclische Verbindungen dar. Letztere sind in einfacher Weise durch Umsetzung von sekundären cyclischen Aminen mit geeignet substituierten epoxifunktionellen Halogenalkanen, und anschließendem Anionenaustausch zugänglich.

Bevorzugt können X und Y in Formel I unabhängig voneinander für ggf. substituierte Alkylengruppen stehen, wobei C₄-C₆- Alkylenketten insbesondere in beiden N-zentrierten Ringen bevorzugt sind und mindestens einer der beiden Substituenten X und oder Y eine OH-Gruppe trägt. Die C₄-C₆- Alkylenketten sind dabei vorzugsweise linear aufgebaut. Diese sind z.B. in einfacher Weise durch Reaktion von ggf. C-substituierten Pyrrolidinen, Piperidinen und Azepanen (1H-Hexahydroazepinen) mit 1,2-Epoxi-4-halogenbutan, 1,2-Epoxi-5-halogenpentan oder 1,2-Epoxi-6-halogenhexan sowie deren C-substituierter Derivate zugänglich, wobei Halogen für Cl, Br und I, bevorzugt Cl, steht.

Weiterhin sind beispielsweise durch analoge Umsetzung von ggf. C-substituierten Oxazolidinen, Isoxazolidinen, Oxazinanen, Morpholinen und Oxazepanen sowie den Analoga der vorstehend genannten N-O-Heterocyclen, die S statt O enthalten, weiterhin Imidazolidinen, Pyrazolidinen, Piperazinen und strukturell verwandten Verbindungen mit o.g. epoxifunktionellen Halogenalkanen auch Vertreter zugänglich, die in einem der Segmente X oder Y der allg. Formel I durch Heteroatome unterbrochene C-Ketten aufweisen. Bei den 2 oder mehr Stickstoffatome enthaltenden Spezies besteht darüber hinaus die Möglichkeit, durch entsprechende Variation der Reaktionsbedingungen auch Salze mit zwei- bzw. mehrfach geladenem Kation zu erzeugen oder durch vorherige geeignete Substitution des bzw. der N-Atome zu einfach positiv geladenen Kationen der Formel I zu gelangen in denen sich ein bzw. mehrere exocyclische(r) Alkylsubstituent(en) an dem bzw. den dreibindigen N-Atom(en) des Ringes X bzw Y befindet bzw. befinden.

Natürlich lässt sich auch durch geeignete Wahl des Alkylierungsmittels eine strukturelle Variation in das Ringsegment X oder Y einbringen, beispielhaft seien Reaktionen von 1,2-Epoxi-omegahalogenalkylethern mit den o.g. sekundären Aminen genannt.

Verbindungen der Formel I sind in hohen Ausbeuten durch Addition von sekundären Aminen an Halogenalkyl-oxirane unter simultan ablaufender Ringöffnung des Oxiranringes und Quaternisierung des Stickstoffatomes des vormals sekundären Amins zugänglich.

Dieser Syntheseweg zu zyklischen, quaternären Ammoniumsalzen die eine Hydroxyfunktion in 2-Stellung zum ladungstragenden N-Atom am Ring aufweisen, ist bisher nicht beschrieben.

Daher ist ein weiterer Gegenstand der vorliegenden Erfindung ein Verfahren zur Herstellung der erfindungsgemäßen Katalysatorkomponenten, umfassend die Schritte
a) Umsetzung eines, ggf. cyclischen sekundären Amins R¹,R²NH, in dem R¹ und R² für die gleichen Substituenten wie in Formel I stehen,
b) gegebenenfalls in Gegenwart eines Lösemittels
c) mit epoxifunktionellen Halogenalkanen unter Ausbildung der erfindungsgemäßen quaternären Ammoniumhalogenide die ggf. anschließend in einem Schritt
d) durch Austausch des Halogenatoms in Ammoniumverbindungen mit beliebigem anderen Anion überführt werden können.

Es existiert mit DE 946 708 lediglich eine Vorbeschreibung für die Bildung von ungeladenen beta-Oxypyrrolidinen durch Reaktion von Ammoniak oder primären Aminen mit beta-Halogenethylethylenoxiden, bevorzugt in Gegenwart von Basen. Der Fachmann musste mithin davon ausgehen, dass die Basen-induzierte HCl-Abspaltung aus den möglicherweise als Intermediat gebildeten NHfunktionellen Ammoniumchloriden essenziell für das Ablaufen der Reaktion ist.

Diese Erwartung wird weiter gestützt durch CN 105 151 266A in der die Reaktion von Chloralkyloxiranen mit Piperidin zu Bis-piperidinyl-alkanolen beschrieben ist und keine Cyclisierung unter Quaternierung des Stickstoffatomes beschrieben wurde.

In JP 2012056897 wird die Synthese von zyklischen, quaternären Ammoniumsalzen die eine Hydroxyfunktion am Ring aufweisen durch Reaktion OH-funktioneller, sekundärer, cyclischer Amine mit alpha,omega-Dihalogenalkanen in schlechten Ausbeuten beschrieben. Neben der schlechten Ausbeute ist hierbei von Nachteil, dass die hierfür nötigen OH-funktionellen, sekundären, cyclischen Amine kommerziell deutlich schlechter zugänglich sind als die OH-freien Grundkörper wie Pyrrolidin oder Piperidin während die in der erfindungsgemäßen Synthese einzusetzenden Halogenalkyloxirane durch einfache Oxidation (Epoxidierung) technisch gut verfügbarer bzw. einfach herstellbarer Halogenolefine geeigneter Struktur problemlos zugänglich sind (siehe Ausführungsbeispiel 1 der vorliegenden Erfindung).

Bevorzugt werden Umsetzungsprodukte aus beliebigen sekundären Aminen und 2-(2-halogenethyl)oxiranen (II) bzw. 2-(3-halogenpropyl)oxiranen (III) 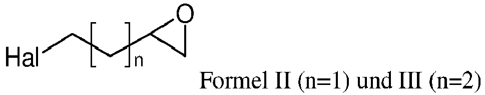 eingesetzt, da die dem organischen Chemiker allgemein bekannte Stabilität von 5- und 6-gliedrigen Ringen hier zu optimalen Resultaten führt. Darüber hinaus sind aber auch größere Zyklen darstellbar wobei ggf. nach dem Ruggli-Zieglerschen Verdünnungsprinzip gearbeitet werden muss. Hal steht in Formel II bzw. III für Chlor, Brom oder Iod, bevorzugt für Chlor oder Brom, besonders bevorzugt für Chlor.

Beispiele für Umsetzungsprodukte mit OH-funktionellem 5-Ring-Segment sind aus 1,2-Epoxi-4-Haloalkanen zugänglich. Nachstehend werden nur die Kationen genannt (IPAC-Namen wurden mit dem Programm BioVia / Draw, MDL.Draw.Editor 16.1.0.693 generiert): 1,1-Dimethylpyrrolidin-1-ium-3-ol, 1,1-Diethylpyrrolidin-1-ium-3-ol, 1,1-Dibutylpyrrolidin-1-ium-3-ol, 5-Azoniaspiro[4.4]nonan-3-ol, 5-Azoniaspiro[4.5]decan-3-ol, 5-Azoniaspiro[4.6]undecan-3-ol, 4,6,6-Trimethylspiro[2-azoniabicyclo[2.2.2]octane-2,1'-azolidin-1-ium]-3'-ol, 8-Oxa-5-azoniaspiro[4.5]decan-3-ol, 8-Methyl-8-aza-5-azoniaspiro[4.5]decan-3-ol, 5,8-Diazoniadispiro[4.2.48.25]tetradecane-3,11-diol.

Beispiele für Umsetzungsprodukte mit OH-funktionellem 6-Ring-Segment (aus 1,2-Epoxi-5-Haloalkanen) sind: 1,1-Dimethylpiperidin-1-ium-3-ol, 1,1-Diethylpiperidin-1-ium-3-ol, 1,1-Dibutylpiperidin-1-ium-3-ol, 5-Azoniaspiro[4.5]decan-9-ol, 6-Azoniaspiro[5.5]undecan-4-ol, 6-Azoniaspiro[5.6]dodecan-4-ol, 4,6,6-Trimethylspiro[2-azoniabicyclo[2.2.2]octane-2,1'-azinan-1-ium]-3'-ol, 3-Oxa-6-azoniaspiro[5.5]undecan-10-ol, 3-Methyl-3-aza-6-azoniaspiro[5.5]undecan-10-ol sowie 6,9-Diazoniadispiro[5.2.59.26]hexadecane-4,13-diol.

Als Anionen können bei den Verbindungen der Formel I prinzipiell alle Strukturtypen zum Einsatz kommen, die als katalytisch aktiv gegenüber Isocyanaten bekannt sind, bevorzugt sind das Hydroxid, Alkanoat, Carboxylat, Heterocyclen mit mindestens einem negativ geladenen Stickstoffatom im Ring, insbesondere Azolat, Imidazolat, Triazolat, Tetrazolat, Fluorid, Hydrogendifluorid, höhere Polyfluoride oder Mischungen von diesen (Addukte von mehr als einem Äquivalent HF an Fluorid-Ionen enthaltenden Verbindungen), wobei die Fluoride, Hydrogendifluoride und höheren Polyfluoride erfindungsgemäß zu Produkten mit hohem Iminooxadiazindiongruppengehalt führen.

Die erfindungsgemäße Katalysatorkomponente ist besonders für die Isocyanatmodifizierung geeignet, da sie insbesondere dort eine hohe katalytische Aktivität und Selektivität bei gleichzeitig guter Katalysatorstabilität und geringer Neigung zur Bildung störender Nebenprodukte zeigt. Ein weiterer Gegenstand der vorliegenden Erfindung ist daher ein Verfahren zur Modifizierung von Isocyanaten, bei dem mindestens ein organisches Isocyanat mit einer NCO-Funktionalität > 1 in Gegenwart eine Katalysatorkomponente oligomerisiert wird, dadurch gekennzeichnet, dass die Katalysatorkomponente mindestens ein cyclisches Ammoniumsalz mit einem Kation der Formel I, 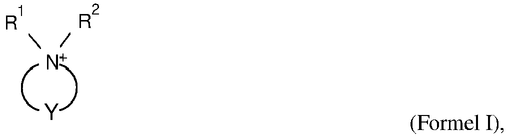 wobei
Y für ein in 2-Stellung zum ladungstragenden Stickstoffatom durch eine Hydroxygruppe substituiertes und ggf. weitere Substituenten tragendes, lineares oder verzweigtes, ggf. durch Heteroatome aus der Reihe Sauerstoff, Schwefel, Stickstoff sowie aromatische Ringe unterbrochenes, ggf. weitere Ringe aufweisendes, C₂-C₂₀-Segment steht und
die N-ständigen Substituenten R¹ und R² entweder unabhängig voneinander für gleiche oder verschiedene, substituierte oder unsubstituierte, ggf. verzweigte, aliphatische C₁-C₂₀ Reste, aromatische C₆-C₂₀ Reste oder araliphatische C₇-C₂₀ Reste stehen oder
die N-ständigen Substituenten R¹ und R² untereinander ein Ringsegment X bilden, für das die gleiche oder verschiedene vorstehend für Y gegebene Definition gilt, mit der Maßgabe, dass X eine in 2-Stellung zum ladungstragenden Stickstoffatom stehende Hydroxygruppe als Substituent aufweist oder keine in 2-Stellung zum ladungstragenden Stickstoffatom stehende Hydroxygruppe als Substituent aufweist. Die vorstehend für die Katalysatorkomponente als bevorzugt genannten Ausführungsformen des Kations der Formel I sind auch bei dem erfindungsgemäßen Verfahren bevorzugte Ausführungsformen.

Die erfindungsgemäßen Katalysatoren können einzelnen oder in beliebigen Mischungen untereinander verwendet werden. So liegen die Lösungen quaternärer Ammoniumhydroxide in verschiedenen Alkoholen je nach pKs-Wert der Base und des verwendeten Alkohols teilweise oder vollständig als Ammoniumsalze mit Alkoholat-Anion vor. Dieses Gleichgewicht kann durch Entfernung des aus dieser Reaktion resultierenden Reaktionswassers ganz auf die Seite vollständiger Alkoholatbildung verschoben werden, wobei im Einzelfall auch die ringgebundene OH-Funktion hierbei involviert sein kann (Betainbildung). Als Methoden zur Wasserentfernung eignen sich dabei alle aus der Literatur hierfür bekannten Methoden, insbes. die Azeotropdestillation ggf. unter Zuhilfenahme eines geeigneten Schleppmittels.

Bei dem erfindungsgemäßen Verfahren kann weiter vorgesehen sein, dass die Oligomerisierung unter Anwesenheit eines Lösungsmittels durchgeführt wird.

Zur Durchführung des erfindungsgemäßen Verfahrens können prinzipiell alle bekannten Mono-, Di- oder Polyisocyanate des Standes der Technik einzeln oder in beliebigen Mischungen untereinander eingesetzt werden.

Beispielhaft seien genannt: Pentamethylendiisocyanat (PDI), Hexamethylendiisocyanat (HDI), 2-Methylpentan-1,5-diisocyanat (MPDI), 2,4,4-Trimethyl-1,6-hexandiisocyanat und 2,2,4-Trimethyl-1,6-hexandiisocyanat (TMDI), 4-Isocyanatomethyl-1,8-octandiisocyanat (Nonantriisocyanat, NTI), 3(4)-Isocyanatomethyl-1-methylcyclohexylisocyanat (IMCI), Isophorondiisocyanat (IPDI), 1,3-sowie 1,4-Bis(isocyanatomethyl)benzen (XDI), 1,3- sowie 1,4-Bis(isocyanatomethyl)cyclohexan (H₆XDI), Norbornandiisocyanat (NBDI), 2,4- sowie 2,6-Toluylendiisocyanat (TDI), Bis(4-isocyanatophenyl)methan (4,4'MDI), 4-Isocyanatophenyl-2-isocyanatophenylmethan (2,4'MDI) sowie mehrkernige Produkte, die durch Formaldehyd-Anilin-Polykondensation und anschließende Überführung der resultierenden (Poly)amine in die entsprechenden (Poly)isocyanate zugänglich sind (Polymer-MDI).

Bevorzugt sind aromatische Diisocyanate, d.h. Diisocyanate bei denen beide NCO-Gruppen an ein sp²-hybridisiertes Kohlenstoffatom gebunden sind, oder aliphatische Diisocyanate, d.h. Diisocyanate bei denen beide NCO-Gruppen an ein sp³-hybridisiertes Kohlenstoffatom gebunden sind.

Besonders bevorzugt sind PDI, HDI, MPDI, TMDI, NTI, IPDI, IMCI, XDI, H₆XDI, MDI, TDI oder NBDI.

Es ist belanglos, nach welchen Verfahren die vorstehend genannten Isocyanate generiert werden, d.h. mit oder ohne Verwendung von Phosgen.

Die Menge des im erfindungsgemäßen Verfahren einzusetzenden Katalysators richtet sich in erster Linie nach dem verwendeten organischen Isocyanat und der angestrebten Reaktionsgeschwindigkeit und liegt vorzugsweise zwischen ≥ 0,001 und ≤ 5 mol-%, bevorzugt zwischen ≥ 0,002 und ≤ 2 mol-%, bezogen auf die Summe der Stoffmengen des eingesetzten Isocyanates und des Katalysators.

Der Katalysator kann im erfindungsgemäßen Verfahren unverdünnt oder in Lösungsmitteln gelöst eingesetzt werden. Als Lösungsmittel kommen dabei alle Verbindungen in Frage, die nicht mit dem Katalysator reagieren und ihn in ausreichendem Maße zu lösen vermögen, z.B. ggf. halogenierte, aliphatische oder aromatische Kohlenwasserstoffe, Alkohole, Ketone, Ester sowie Ether. Bevorzugt werden Alkohole verwendet.

Das erfindungsgemäße Verfahren kann im Temperaturbereich von 0 °C bis + 250 °C, bevorzugt 20 °C bis 200 °C, besonders bevorzugt 40 °C bis 150 °C erfolgen und bei beliebigen Umsetzungsgraden, bevorzugt nachdem 5 bis 80 %, besonders bevorzugt 10 bis 60 %, des eingesetzten Isocyanates umgesetzt wurden, unterbrochen werden.

Zur Katalysatordeaktivierung bieten sich prinzipiell eine ganze Reihe vorbeschriebener Methoden des Standes der Technik an, wie z.B. die Zugabe (unter- oder über-) stöchiometrischer Mengen an Säuren oder Säurederivaten (z.B. Benzoylchlorid, saure Ester Phosphor oder Schwefel enthaltender Säuren, diese Säuren selbst etc., nicht jedoch HF), adsorptive Bindung des Katalysators und anschließende Abtrennung durch Filtration und andere dem Fachmann bekannte Methoden.

In einer weiteren bevorzugten Ausführungsform wird nicht umgesetztes organisches Isocyanat nach Desaktivierung des Katalysatorsystems nach einem beliebigen Verfahren des Standes der Technik, z.B. durch (Dünnschicht)destillation oder Extraktion, abgetrennt und bevorzugt anschließend wiederverwendet.

Im Gegensatz zur Katalyse mit offenkettigen, hydroxyfunktionellen Ammoniumsalzen in denen das ladungstragende Stickstoffatom nicht Teil eines Ringsystems ist (vgl. z.B. EP 10589), beobachtet man bei Einsatz der erfindungsgemäßen Katalysatoren überraschenderweise auch bei höheren Reaktionstemperaturen kein nennenswertes Nachlassen der katalytischen Aktivität.

Ganz allgemein sind die erfindungsgemäßen Katalysatoren unabhängig vom Anion, das für die katalytische Aktivität und Selektivität verantwortlich ist, im umzusetzenden organischen Isocyanat wesentlich stabiler als die literaturbekannten Derivate des Standes der Technik.

Nach einer besonderen, kontinuierlich arbeitenden Ausführungsform des erfindungsgemäßen Verfahrens kann die Oligomerisierung kontinuierlich, z.B. in einem Rohrreaktor vorgenommen werden.

Mit dem erfindungsgemäßen Modifizierverfahren ist ganz allgemein eine breite Palette qualitativ hochwertiger und deshalb für den Polyurethansektor sehr wertvoller modifizierter Isocyanate, vorliegend auch als Polyisocyanate bezeichnet, in einfacher Weise zugänglich. Ein weiterer Gegenstand der vorliegenden Erfindung ist ein modifiziertes Isocyanat, erhältlich oder hergestellt durch das erfindungsgemäße Verfahren. Die erfindungsgemäße Katalysatorkomponente reagiert über die OH-Gruppe unter Ausbildung einer kovalenten Bindung in das modifizierte Isocyanat ein. Somit ist ebenfalls Gegenstand der vorliegenden Erfindung ein modifiziertes Isocyanat, enthaltend mindestens ein über die OH-Gruppe des Kations der Formel I aus der erfindungsgemäßen Katalysatorkomponente als Urethan- und/oder Allophanatgruppe kovalent gebundenes Strukturelement. Die vorstehend für die Katalysatorkomponente als bevorzugt genannten Ausführungsformen des Kations der Formel I sind auch bei dem erfindungsgemäßen modifizierten Isocyanat bevorzugte Ausführungsformen.

Abhängig vom verwendeten Ausgangs(di)isocyanat und den Reaktionsbedingungen entstehen beim erfindungsgemäßen Verfahren Polyisocyanate vom sog. Isocyanat-Trimertyp (d.h. enthaltend Isocyanurat- und/oder Iminooxadiazindionstrukturen) mit geringem Anteil an Uretdiongruppen ("Isocyanat-Dimere"). Bei steigender Reaktionstemperatur steigt der Anteil letzterer in den Verfahrensprodukten in der Regel an.

Die nach dem erfindungsgemäßen Verfahren erhältlichen Produkte bzw. Produktgemische stellen mithin vielseitig verwendbare Ausgangsmaterialien zur Herstellung von, ggf. geschäumte(n), Kunststoffe(n) sowie Lacken, Beschichtungsmitteln, Klebstoffen und Zuschlagstoffen dar. Daher ist die Verwendung der erfindungsgemäßen modifizierten Isocyanate zur Herstellung von geschäumten oder ungeschäumten Kunststoffen sowie Lacken, Beschichtungsmitteln, Klebstoffen und Zuschlagstoffen ein weiterer Gegenstand der vorliegenden Erfindung. Weiterhin sind die erfindungsgemäßen Katalysatoren für die Herstellung ggf. geschäumter Polyurethankörper unter anteiliger Polyisocyanuratbildung (sog. PIR-Schäume) gut geeignet, da sie praktisch nicht aus den Fertigprodukten migrieren können, was unter dem Aspekt der Migrationsfreiheit (leaching, fogging) von Bedeutung ist. Folglich sind Polyurethankörper, erhältlich oder hergestellt durch Umsetzung wenigstens eines monomeren Diisocyanats und/oder Polyisocyanats mit wenigstens einer Polyolkomponente in Gegenwart der erfindungsgemäßen Katalysatorkomponente ein weiterer Gegenstand der Erfindung. Für den Fall, dass es sich um geschäumte Polyurethankörper handelt, sind es bevorzugt PIR-Schäume.

Die erfindungsgemäßen Verfahrensprodukte können als solche oder in Verbindung mit anderen Isocyanatderivaten des Standes der Technik, wie z.B. Uretdion-, Biuret-, Allophanat-, Isocyanurat- und/oder Urethan-Gruppen enthaltenden Polyisocyanaten, deren freie NCO-Gruppen ggf. mit Blockierungsmitteln desaktiviert wurden, eingesetzt werden.

Weitere Gegenstände der vorliegenden Erfindung sind ein Ein- oder Zweikomponenten-Systeme, enthaltend eine Komponente A), umfassend mindestens ein erfindungsgemäßes modifiziertes Isocyanat, und eine Komponente B), umfassend mindestens eine NCO-reaktive Verbindung, sowie eine Beschichtung, erhältlich oder hergestellt durch, ggf. unter Wärmeeinwirkung und/oder in Anwesenheit eines Katalysators, Härten eines erfindungsgemäßen Ein- oder Zwei-Komponenten-Systems aber auch die mit mindestens einem erfindungsgemäßen, gegebenenfalls unter Wärmeeinwirkung ausgehärteten, Ein- oder Zweikomponenten-System, beschichteten Substrate. Da sich die erfindungsgemäßen modifizierten Isocyanate über die kovalent eingebundene erfindungsgemäße Katalysatorkomponente auch in den gehärteten oder geschäumten Polyurethankörpern wiederfinden, ist ein Verbundbauteil, umfassend einen Werkstoff, der mit einem erfindungsgemäßen Polyurethankörper oder einer erfindungsgemäßen Beschichtung zumindest anteilig verbunden ist, ebenfalls Gegenstand der Erfindung.

Vorliegend hat der Begriff "modifiziertes Isocyanat" die eingangs definierte Bedeutung und steht vorzugsweise für ein Polyisocyanat, welches im statistischen Mittel mindestens 1,5 NCO-Gruppen aufweist.

Die nachfolgenden Vergleichsbeispiele und Beispiele sollen die Erfindung näher erläutern, ohne sie jedoch einzuschränken.

### Beispiele:

Alle Prozentangaben sind, soweit nicht anders vermerkt, als Gewichtsprozent zu verstehen.

Mol-% Angaben wurden NMR-spektroskopisch ermittelt und beziehen sich immer, so nicht anders ausgewiesen, auf die Summe der NCO-Folgeprodukte. Die Messungen erfolgten auf den Geräten DPX 400 bzw. DRX 700 der Fa. Brucker an ca. 5 %igen (¹H-NMR) bzw. ca. 50 %igen (¹³C-NMR) Proben in trockenem C₆D₆ bei einer Frequenz von 400 bzw. 700 MHz (¹H-NMR) oder 100 bzw. 176 MHz (¹³C-NMR). Als Referenz für die ppm-Skale wurden geringe Mengen von Tetramethylsilan im Lösungsmittel mit 0 ppm ¹H-NMR-chem. Verschiebung herangezogen. Alternativ wurde auf das Signal des im Lösungsmittel enthaltenen C₆D₅H referenziert: 7,15 ppm ¹H-NMR-chem. Verschiebung, 128,02 ppm ¹³C-NMR-chem. Verschiebung. Daten für die chemische Verschiebung der in Frage kommenden Verbindungen wurden der Literatur entnommen (vgl. D. Wendisch, H. Reiff und D. Dieterich, Die Angewandte Makromolekulare Chemie 141, 1986, 173-183 und darin zit. Lit sowie EP 896 009 A1).

Die dynamischen Viskositäten wurden bei 23 °C mit dem Viskosimeter VT 550 der Fa. Haake nach der DIN EN ISO 3219:1994-10 bestimmt. Durch Messungen bei unterschiedlichen Schergeschwindigkeiten wurde sichergestellt, daß das Fließverhalten der beschriebenen erfindungsgemäßen Polyisocyanatmischungen wie auch das der Vergleichsprodukte dem idealer Newtonscher Flüssigkeiten entspricht. Die Angabe der Schergeschwindigkeit kann deshalb entfallen.

Die Ermittlung des NCO-Gehaltes erfolgte durch Titration gemäß DIN EN ISO 11909:2007-05.

Die Bestimmung der Restmonomergehalte erfolgte gaschromatographisch nach der DIN EN ISO 10283:2007-11 mit internem Standard.

Alle Reaktionen wurden, wenn nicht anders angegeben, unter einer Stickstoffatmosphäre durchgeführt.

Die verwendeten Diisocyanate sind Produkte der Covestro AG, D-51365 Leverkusen, alle anderen kommerziell zugänglichen Chemikalien wurden von der Fa. Aldrich, D-82018 Taufkirchen, bezogen.

Die nicht kommerziell erhältlichen Edukte wurden nach literaturbekannten Methoden gewonnen.

Beispielhaft beschrieben sei hier die Synthese der Katalysatoren 1 und 2 nach folgender Reaktionssequenz:

### Beispiel 1: Herstellung von Katalysator 1 und 2

10 g 3-Buten-1-ol (ca. 0,14 mol) wurden unter Rühren (Magnetrührer) in einem 100 ml Zweihalskolben bei Zimmertemperatur vorgelegt, mit 7 Tropfen (ca. 50 mg; ca. 0,6 mmol) Pyridin versetzt und durch Eintauchen in ein Eis-Wasser-Gemisch auf ca. 0°C gekühlt. Anschließend wurden 17,8 g Thionylchlorid (ca. 0,15 mol) langsam unter Rühren (Magnetrührer) zugetropft wobei unter lebhafter Gasentwicklung leichte Gelbfärbung eintrat. Anschließend wurde 6 h bei 80°C nachgerührt, die nun gelbbraune Mischung zügig bei leicht vermindertem Druck in eine Kältevorlage (-78°C, p anfangs 700 mbar, stufenweise bis 200 mbar abgesenkt) destilliert und das dabei anfallende Destillat anschließend bei Normaldruck langsam über eine 10 cm lange Vigreuxkolonne fraktioniert. Das 1-Chlor-3-buten siedete bei 75 +/- 1°C. Es wurden 9,4 g (ca. 0,1 mol; ca. 75% Ausbeute, nicht optimiert) als farblose Flüssigkeit erhalten.

Diese Menge an 1-Chlor-3-buten wurde anschließend in ca. 150 ml Methylenchlorid gelöst und portionsweise unter kräftigem mechanischem Rühren summarisch ca. 25 g Metachlorperbenzoesäure (77%ige Reinheit lt. Herstellerangabe) eingetragen. Nach 24 stündigem Rühren bei Zimmertemperatur wurde filtriert, der Filterrückstand 3 mal mit je ca. 100 ml Methylenchlorid gewaschen, die vereinigten Filtrate zügig bei leicht vermindertem Druck in eine Kältevorlage (-78°C, p anfangs 700 mbar, stufenweise bis 20 mbar abgesenkt) und das dabei anfallende Destillat anschließend langsam über eine 10 cm lange Vigreuxkolonne zunächst bei Normaldruck bis zur nahezu restlosen Entfernung des Methylenchlorides und dann weiter bei 50 mbar fraktioniert. Das 1-Chlor-3,4-butenoxid siedete bei 70 +/- 2°C. Es wurden 8,5 g (ca. 0,08 mol; ca. 80% Ausbeute, nicht optimiert) als farblose Flüssigkeit erhalten.

Diese Menge an 1-Chlor-3,4-butenoxid wurde unter Rühren (Magnetrührer) zu einer am Rückfluß siedenden Mischung aus 6,8 g Piperidin (ca. 0,08 mol) und ca. 150 g Wasser getropft, eine halbe Stunde bei 120°C Badtemperatur nachgerührt, unter vermindertem Druck von allen flüchtigen Bestandteilen befreit und der verbleibende Rückstand ohne weitere Reinigung (Ausbeute nahezu quantitativ) geteilt und durch a) Zugabe eines Aliquotes methanolische KOH-Lösung in das Hydroxid und b) Zugabe eines 100%igen Überschusses an methanolischer KF-Lösung in das Fluorid überführt.

Die unter a) erhaltene Lösung wurden filtriert, der Niederschlag mehrfach mit 2-Ethylhexanol (im weiteren Text: 2-EH) gewaschen und nach Einengen (MeOH-Freiheit mittels ¹H-NMR überprüft) auf den gewünschten Gehalt an Katalysator 1 eingestellt (s. Tabelle 1).

Die unter b) erhaltene Lösung wurden filtriert, der Niederschlag mehrfach mit 2-EH gewaschen und nach Einengen (MeOH-Freiheit mittels ¹H-NMR überprüft) mit 1,2 Equivalenten einer 10%igen Lösung von wasserfreiem HF in 2-EH und anschließender Verdünnung mit weiterem 2-EH auf den gewünschten Gehalt an Katalysator 2 eingestellt (s. Tabelle 1).

Völlig analog mit vergleichbaren Ausbeuten ist die Synthese weiterer Spezies ausgehend von 4-Penten-1-ol einerseits sowie anderen sekundären Aminen wie Pyrrolidin oder Dimethylamin (es wurde die wässrige Lösung eingesetzt und unter leichtem Überdruck gearbeitet) andererseits, möglich.

Weitere Katalysatoren wurden nach analogen Verfahren aus den entsprechenden quaternären Ammoniumchloriden gewonnen, welche ihrerseits vorher aus dem jeweiligen sekundären, ggf. cyclischen Amin und dem entsprechenden Chloralkyloxiran dargestellt worden war. Höhere Oligofluoride wurden durch Zugabe entsprechender HF-Überschüsse zu den in Analogie zu Katalysator 2 erhaltenen (Poly)Fluoridlösungen erhalten. Andere Anionen als Hydroxid oder (Poly)fluorid wurden durch Salzmethathese mit beispielsweise Kaliumacetat oder Kaliumpivalat gewonnen.

Die optimale Katalysatorkonzentration für die Isocyanat-Trimerisierung wurde in orientierenden Vorversuchen bei 60 °C mit HDI (vgl. Beispiel 2) ermittelt und die Konzentration der Katalysatorlösung durch Verdünnen mit 2-EH so eingestellt, dass keine bzw. nur vernachlässigbar geringe Gelteilchenbildung bei der Zugabe der Katalysatorlösung zum HDI zu beobachten war. Eine Übersicht der verwendeten Katalysatoren findet sich in Tabelle 1.

**Tabelle 1: Übersicht der hergestellten Katalysatoren (die Katalysatorkonzentration bezieht sich auf den Wirkstoff (Kation und Anion))**

| Katalysator | Kation | Anion | Konzentration [%] |
|---|---|---|---|
| 1 | 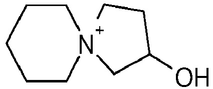 | OH⁻ | 2 |
| 5-Azoniaspiro[4.5] decan-3-ol-hydroxid | | | |
| 2 | 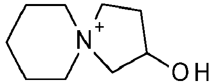 | [F*1,2 HF]⁻ | 25 |
| 5-Azoniaspiro[4.5] decan-3-ol fluorid * 1,2 HF | | | |
| 3 | 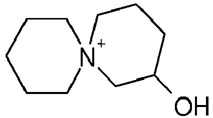 | (CH₃)₃CC(O)O⁻ | 10 |
| 6-Azoniaspiro[5.5] undecan-4-ol | | | |
| 4 | 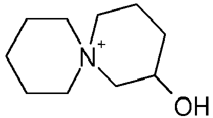 | [HF₂]⁻ | 30 |
| 6-Azoniaspiro[5.5] undecan-4-ol hydrogendifluorid | | | |
| 5 | 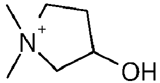 | OH⁻ | 1 |
| 1,1-Dimethylpyrrolidin-1-ium-3-ol hydroxid | | | |
| 6 | 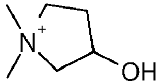 | [HF₂]⁻ | 10 |
| 1,1-Dimethylpyrrolidin-1-ium-3-ol hydrogendifluorid | | | |
| 7 | 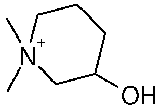 | CH₃C(O)O⁻ | 10 |
| 1,1-Dimethylpiperidin-1-ium-3-ol pivalat | | | |
| 8 | 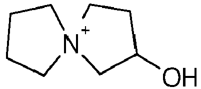 | OH⁻ | 1 |
| 5-Azoniaspiro[4.4] nonan-3-ol hydroxid | | | |
| 9 | 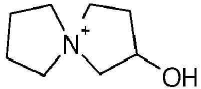 | [HF₂]⁻ | 15 |
| 5-Azoniaspiro[4.4] nonan-3-ol hydrogendifluorid | | | |

### Beispiele 2 bis 9 - erfindungsgemäße Isocyanat-Modifizierungen

In einem doppelwandigen, durch einen externen Kreislauf auf die jeweils gewünschte Starttemperatur temperierten Planschliffgefäß mit Rührer, an eine Inertgasanlage (Stickstoff/Vakuum) angeschlossenem Rückflusskühler und Thermometer wurden 1000 g HDI vorgelegt und durch einstündiges Rühren im Vakuum (< 1 mbar) von gelösten Gasen befreit. Nach Belüften mit Stickstoff wurde die in Tabelle 2 angegebene Katalysatorart und Katalysatormenge ggf. portionsweise so dosiert, dass die in Tabelle 2 angegebene Maximaltemperatur nicht überschritten wurde. Nachdem ca. 1 mol NCO Gruppen umgesetzt waren, indiziert durch Erreichen eines NCO-Gehaltes um 45,8 %, wurde der Katalysator durch Zugabe einer zum Katalysator äquivalenten Menge des in Tabelle 2 angegebenen Stoppers deaktiviert, weitere 30 min bei Reaktionstemperatur nachgerührt und anschließend aufgearbeitet.

Die Aufarbeitung erfolgte durch Vakuumdestillation in einem Dünnschichtverdampfer, Typ Kurzwegverdampfer (KWV), mit vorgeschaltetem Vorverdampfer (VV) (Destillationsdaten: Druck: 0,08 +/- 0,04 mbar, VV-Temperatur: 120 °C, HV-Temp.: 140 °C), wobei nicht umgesetztes Monomer als Destillat und das monomerenarme Polyisocyanatharz als Sumpfprodukt separiert wurden (Startdurchlauf). Das Polyisocyanatharz wurde separiert und das Destillat in einer zweiten Planschliff-Rührapparatur, die identisch zur ersten aufgebaut ist, gesammelt und mit frisch entgastem HDI auf die Ausgangsmenge (1000 g) aufgefüllt. Anschließend wurde erneut katalysiert und verfahren wie eingangs beschrieben. Diese Verfahrensweise wurde, ggf. unter Variation der Reaktionstemperatur, mehrmals wiederholt (Versuche A, B, C, usw.). Die Ergebnisse sind Tabelle 2 zu entnehmen.

Abschließend wurde die Destillatzusammensetzung gaschromatografisch ermittelt. In keinem Fall konnten Zersetzungsprodukte des Katalysatorkations detektiert werden (Nachweisgrenze ca. 20 ppm).

**Tabelle 2: Durchgeführte Isocyanatmodifizierungen**

| Beispiel | | Katalysator (s. Tabelle 1) | Katalysatormenge [g] | Reaktionstemperatur von - bis [°C] | | Stopper* |
|---|---|---|---|---|---|---|
| 1 | A | 1 | 4,3 | 60 | 62 | 1 |
| 1 | B | 1 | 5,7 | 60 | 71 | 1 |
| 1 | C | 1 | 6,2 | 80 | 90 | 1 |
| 1 | D | 1 | 5,9 | 80 | 85 | 1 |
| 1 | E | 1 | 6,5 | 100 | 120 | 1 |
| 1 | F | 1 | 6,8 | 100 | 140 | 1 |
| 2 | A | 2 | 0,85 | 60 | 64 | 3 |
| 2 | B | 2 | 0,81 | 60 | 63 | 3 |
| 2 | C | 2 | 0,75 | 80 | 88 | 3 |
| 2 | D | 2 | 0,61 | 80 | 83 | 3 |
| 2 | E | 2 | 0,62 | 100 | 125 | 3 |
| 2 | F | 2 | 0,61 | 100 | 104 | 3 |
| 3 | A | 3 | 1,42 | 60 | 60 | 1 |
| 3 | B | 3 | 1,82 | 60 | 64 | 1 |
| 3 | C | 3 | 2,11 | 80 | 85 | 1 |
| 3 | D | 3 | 2,00 | 80 | 84 | 1 |
| 3 | E | 3 | 2,10 | 100 | 137 | 1 |
| 3 | F | 3 | 2,22 | 100 | 101 | 1 |
| 4 | A | 4 | 0,91 | 60 | 61 | 2 |
| 4 | B | 4 | 0,95 | 60 | 62 | 2 |
| 4 | C | 4 | 0,69 | 60 | 61 | 2 |
| 4 | D | 4 | 0,67 | 60 | 61 | 2 |
| 4 | E | 4 | 0,60 | 60 | 61 | 2 |
| 4 | F | 4 | 0,58 | 60 | 61 | 2 |
| 5 | A | 5 | 8,5 | 60 | 77 | 1 |
| 5 | B | 5 | 9,2 | 60 | 62 | 1 |
| 5 | C | 5 | 9,8 | 80 | 88 | 1 |
| 5 | D | 5 | 9,5 | 80 | 92 | 1 |
| 5 | E | 5 | 10,2 | 100 | 124 | 1 |
| 5 | F | 5 | 10,9 | 100 | 103 | 1 |
| 6 | A | 6 | 1,71 | 60 | 62 | 2 |
| 6 | B | 6 | 1,42 | 60 | 61 | 2 |
| 6 | C | 6 | 1,25 | 80 | 85 | 2 |
| 6 | D | 6 | 1,02 | 80 | 83 | 2 |
| 6 | E | 6 | 0,95 | 100 | 102 | 2 |
| 6 | F | 6 | 0,98 | 100 | 105 | 2 |
| 7 | A | 7 | 1,32 | 60 | 62 | 1 |
| 7 | B | 7 | 1,65 | 60 | 61 | 1 |
| 7 | C | 7 | 1,98 | 80 | 83 | 1 |
| 7 | D | 7 | 1,98 | 80 | 82 | 1 |
| 7 | E | 7 | 1,89 | 100 | 104 | 1 |
| 7 | F | 7 | 2,01 | 100 | 104 | 1 |
| 8 | A | 8 | 8,7 | 60 | 62 | 1 |
| 8 | B | 8 | 9,4 | 60 | 61 | 1 |
| 8 | C | 8 | 10,1 | 80 | 83 | 1 |
| 8 | D | 8 | 10,2 | 80 | 82 | 1 |
| 8 | E | 8 | 11,0 | 100 | 104 | 1 |
| 8 | F | 8 | 10,8 | 100 | 104 | 1 |
| 9 | A | 9 | 1,12 | 60 | 62 | 3 |
| 9 | B | 9 | 1,20 | 60 | 61 | 3 |
| 9 | C | 9 | 1,02 | 80 | 83 | 3 |
| 9 | D | 9 | 0,98 | 80 | 82 | 3 |
| 9 | E | 9 | 0,82 | 100 | 104 | 3 |
| 9 | F | 9 | 0,84 | 100 | 104 | 3 |

| | | | | | | |
|---|---|---|---|---|---|---|
| * Stopper: 1: Dibutylphosphat, 2: Toluolsulfonsäure, 40 %ig in 2-PrOH, 3: Dodecylbenzolsulfonsäure, 70 %ig in 2-PrOH | | | | | | |

Bei den erhaltenen Harzen handelte es sich ausnahmslos um farbhelle, klare, viskose Flüssigkeiten ohne wahrnehmbaren Amingeruch und ohne extrahierbare Katalysatorneben- oder folgeprodukte. Bei Einsatz der Fluor enthaltenden Katalysatoren resultierten Gemische aus Isocyanurat und Iminooxadiazindion neben wenig Uretdion. Die Katalysatoren mit Sauerstoff-enthaltenden Anionen liefern Produkte vom Isocyanurattyp wobei, wie auch bei Einsatz der Fluor enthaltenden Katalysatoren, der als Katalysatorlösemittel eingesetzte Alkohol (2-EH) vollständig zum Allophanat umgesetzt wird.

Das rückgewonnene HDI ist frei von Verunreinigungen die aus der Zersetzung des Katalysatorkations resultieren und kann problemlos im gleichen oder in anderen Verfahren wieder eingesetzt werden.

## Patentansprüche

1. Katalysatorkomponente für die Isocyanatmodifizierung, umfassend mindestens ein cyclisches Ammoniumsalz mit einem Kation der Formel I, 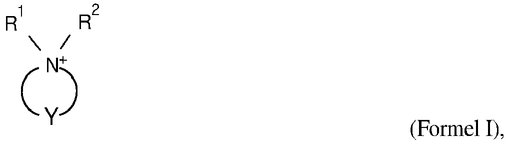
wobei Y für ein in 2-Stellung zum ladungstragenden Stickstoffatom durch eine Hydroxygruppe substituiertes und ggf. weitere Substituenten tragendes, lineares oder verzweigtes, ggf. durch Heteroatome aus der Reihe Sauerstoff, Schwefel, Stickstoff sowie aromatische Ringe unterbrochenes, ggf. weitere Ringe aufweisendes, C₂-C₂₀-Segment steht und
die N-ständigen Substituenten R¹ und R² entweder unabhängig voneinander für gleiche oder verschiedene, substituierte oder unsubstituierte, ggf. verzweigte, aliphatische C₁-C₂₀ Reste, aromatische C₆-C₂₀ Reste oder araliphatische C₇-C₂₀ Reste stehen oder
die N-ständigen Substituenten R¹ und R² untereinander ein Ringsegment X bilden, für das die gleiche oder verschiedene vorstehend für Y gegebene Definition gilt, mit der Maßgabe, dass X eine in 2-Stellung zum ladungstragenden Stickstoffatom stehende Hydroxygruppe als Substituent aufweist oder keine in 2-Stellung zum ladungstragenden Stickstoffatom stehende Hydroxygruppe als Substituent aufweist.

2. Katalysatorkomponente gemäß Anspruch 1, **dadurch gekennzeichnet, dass** Y für ein in 2-Stellung zum ladungstragenden Stickstoffatom durch eine Hydroxygruppe substituiertes und ggf. weitere Substituenten tragendes C₄-C₆-Alkylenkettensegment steht.

3. Katalysatorkomponente gemäß Anspruch 1 oder 2, **dadurch gekennzeichnet, dass** R¹ und R² unabhängig voneinander für gleiche oder verschiedene C₁-C₈-Alkylsubstituenten oder für gleiche oder verschiedene, ggf. am aromatischen Kern substituierte, Benzyl-Reste stehen, bevorzugt für gleiche oder verschiedene C₁-C₆-Alkylsubstituenten stehen und besonders bevorzugt für gleiche oder verschiedene, linear aufgebaute C₁-C₆-Alkylsubstituenten stehen.

4. Katalysatorkomponente gemäß Anspruch 1 oder 2, **dadurch gekennzeichnet, dass** R¹ und R² untereinander ein Ringsegment X bilden, wobei X ein ggf. weitere Substituenten tragendes C₄-C₆-Alkylenkettensegment ist, mit der Maßgabe, dass X in 2-Stellung zum ladungstragenden Stickstoffatom durch eine Hydroxygruppe substituiert ist oder nicht durch eine Hydroxygruppe in 2-Stellung zum ladungstragenden Stickstoffatom substituiert ist.

5. Katalysatorkomponente gemäß einem der Ansprüche 1 bis 4, **dadurch gekennzeichnet, dass** das Segment Y und/oder das Ringsegment X linear aufgebaut sind.

6. Katalysatorkomponente gemäß einem der Ansprüche 1 bis 5, **dadurch gekennzeichnet, dass** nur Y eine Hydroxygruppe aufweist.

7. Verfahren zur Modifizierung von Isocyanaten, bei dem mindestens ein organisches Isocyanat mit einer NCO-Funktionalität > 1 in Gegenwart einer Katalysatorkomponente gemäß einem der Ansprüche 1 bis 6 oligomerisiert und/oder polymerisiert wird.

8. Verfahren gemäß Anspruch 7, **dadurch gekennzeichnet, dass** das organische Isocyanat ein organisches Diisocyanat ist und bevorzugt PDI, HDI, MPDI, TMDI, NTI, IPDI, IMCI, XDI, H6XDI, MDI, TDI oder NBDI ist.

9. Modifiziertes Isocyanat, enthaltend mindestens ein über die OH-Gruppe des Kations der Formel I als Urethan- und/oder Allophanatgruppe kovalent gebundenes Strukturelement, wobei das Kation der Formel 1 eine Struktur nach einem der Ansprüche 1 bis 6 aufweist.

10. Verwendung mindestens eines modifizierten Isocyanats gemäß Anspruch 9 zur Herstellung von geschäumten oder ungeschäumten Kunststoffen sowie Lacken, Beschichtungsmitteln, Klebstoffen oder Zuschlagstoffen.

11. Ein- oder Zwei-Komponenten-System, enthaltend eine Komponente A), umfassend mindestens ein modifiziertes Isocyanat nach Anspruch 9, und eine Komponente B), umfassend mindestens eine gegenüber NCO-Gruppen reaktive Verbindung.

12. Polyurethankörper, erhältlich oder hergestellt durch Umsetzung wenigstens eines monomeren Diisocyanats und/oder Polyisocyanats mit wenigstens einer Polyolkomponente in Gegenwart der Katalysatorkomponente nach einem der Ansprüche 1 bis 6.

13. Polyurethankörper gemäß Anspruch 10, **dadurch gekennzeichnet, dass** der Polyurethankörper ein geschäumter Polyurethankörper, bevorzugt ein Polyisocyanurat-Schaum (PIR-Schaum) ist.

14. Beschichtung, erhältlich oder hergestellt durch, ggf. unter Wärmeeinwirkung und/oder in Anwesenheit eines Katalysators, Härten eines Ein- oder Zwei-Komponenten-Systems nach Anspruch 11.

15. Verbundbauteil, umfassend einen Werkstoff, der mit einem Polyurethankörper gemäß Anspruch 12 oder 13 oder einer Beschichtung gemäß Anspruch 14 zumindest anteilig verbunden ist.
